# EUROPEAN PATENT SPECIFICATION

(11) **EP 0 358 345 B1**
(45) Date of publication and mention of the grant of the patent: **19.01.1994**
(21) Application number: 89308234.7
(22) Date of filing: 14.08.1989
(51) Int. Cl.: A61F 2/34

(54) **Screw-threaded acetabular component of hip joint prosthesis**
Mit einem Schraubengewinde versehenes Acetabularteil einer Hüftgelenksprothese
Composant acétabulaire fileté d'une prothèse de l'articulation de la hanche

(30) Priority: 17.08.1988 GB 8819588
(43) Date of publication of application: 14.03.1990
(73) Proprietor: MINNESOTA MINING AND MANUFACTURING COMPANY, St. Paul, Minnesota 55101 (US)
(72) Inventor: Shelley, Philip, Laughton Enlemouthern Dinnington S31 7YL (GB)
(74) Representative: Bowman, Paul Alan

(56) References cited:
- EP-A- 0 179 736
- EP-A- 0 318 679
- WO-A-87/05490
- DE-U- 8 810 783

## Description

This invention relates to a hip joint prosthesis and in particular to the acetabular component of a hip joint prosthesis having a screw-thread for anchorage within the acetabulum.

Prosthesis for the replacement of hip joints are well known. Originally, only the ball-end on the head of the femur could be replaced but it has since proved possible to replace either part of the hip joint i.e. the acetabular socket of the joint or the ball-end on the femur.

Known acetabular cup implants, which form the socket portion of an artificial hip joint, comprise a metal cup shell, which is secured within the acetabulum in the pelvic bone of a patient, and an inner liner of plastics material which provides a spherical bearing surface for receiving the ball portion of the joint. The metal cup shell may be provided with an external thread to facilitate anchorage to the pelvic bone or may be secured by other means such as cement or screws.

Metal cup shells having an external screw thread are widely used and are disclosed, for example, in US-A-3894297 and US-A-4662891, EP-A-179736 and EP-A-65482 and WO-A-87/05490. EP-A-179736 discloses the features of the preamble of claim 1. However it is often difficult to fully insert metal cup shells having self tapping screw threads which is necessary to ensure contact of the entire surface area of the outside of the metal cup with the surface of the acetabulum to provide maximum transfer of forces from the prosthesis to the pelvic bone. The implants often have a tendency to jam and great force is necessary to rotate the implant in either direction which may cause bone fracture.

EP-A-0318679 which forms part of the state of the art in accordance with Article 54(3) EPC discloses an acetabular implant of a hip joint prosthesis comprising a metal cup shell having an external self-tapping thread extending around the circumference of the shell, the thread being divided into a plurality of segments by a plurality of tapping grooves, whereby the thread radius of thread segments progressively decreases from the leading end to the trailing end of a segment.

A self-tapping thread configuration for an acetabular metal cup shell has now been found which reduces the propensity of the implant to jam during insertion.

According to the present invention there is provided an acetabular implant of a hip joint prosthesis comprising a metal cup shell having an external self-tapping thread extending around the circumference of the shell, the thread being divided into a plurality of segments by a plurality tapping grooves in which the thread radius of thread segments progressively decreases from the leading end to the trailing end of a segment and arranged in pairs dividing the thread into alternate long and short segments.

It has been found that one of the causes of an implant jamming upon insertion is due to the visco-elasticity of bone, which deflects away from the cutting edges on the thread segments and consequently binds upon the trailing portion of the thread segments. This problem is reduced by forming thread segments in which the thread radius (as measured from the outside of the thread segment to the longitudinal axis of the metal cup shell) progressively decreases from the leading end to the trailing end of the segment. The decrease is preferably at least 3% of the depth of the thread segment, more preferably 10% and generally not more than 30%. This arrangement also ensures the leading end of a thread segment presents a sharp cutting face towards the bone thereby cutting the bone rather then deflecting the bone.

Preferably the width of each thread segment also progressively decreases from the leading end to trailing end further reducing the tendency of bone to bind on the trailing portion of a thread segment.

A further cause of an implant jamming during insertion is the inadequate provision of tapping grooves. The purpose of the tapping grooves is to provide a space for the bone fragments cut by the thread segments. Thus it is important that the total volume of the tapping grooves is at least equal to the volume of bone debris generated in the self-tapping process otherwise the tapping grooves become blocked with compact bone debris and further insertion of the implant is prevented. The total volume of the tapping grooves of the implant of the invention is preferably equal to or greater than the total volume of all the thread segments.

It has been found that the particular configuration of tapping grooves can have a marked effect on the tendency of the cup shell to jam during insertion. Wide tapping grooves may have a tendency to allow large irregularities in the bone to enter the groove causing the implant to jam with the risk of bone fracture if high forces are applied. The presence of numerous narrow tapping grooves equally spaced can cause similar problems due to the relatively short length of the thread segments. A particularly effective arrangement is to group the tapping grooves into pairs thereby dividing the thread into alternating longer and shorter segments. The longer thread segments are preferably at least 50% longer than the short segments and usually not more than 1800%, most preferably the long thread segments are 500% longer than the short segments.

The invention will now be illustrated with reference to the accompanying drawings in which:

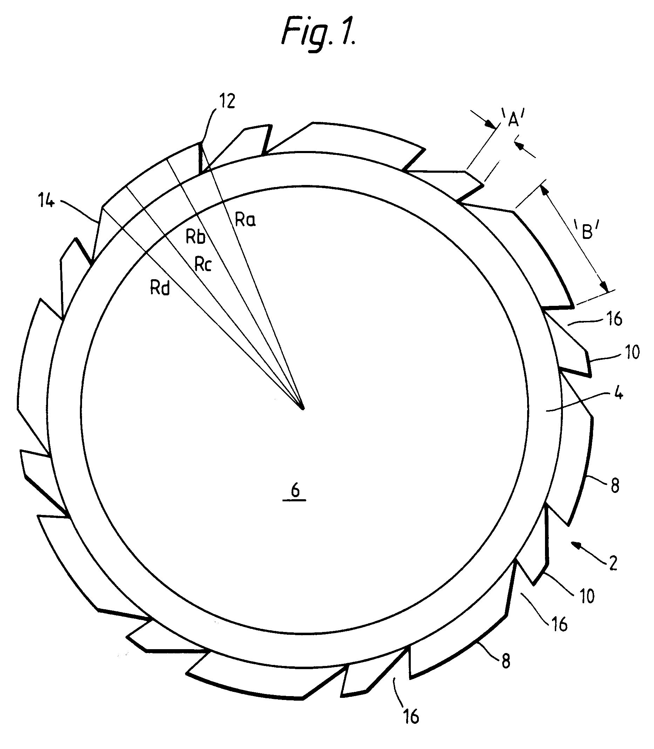

Figure 1 represents a plan view of a metal cup shell in accordance with the invention;

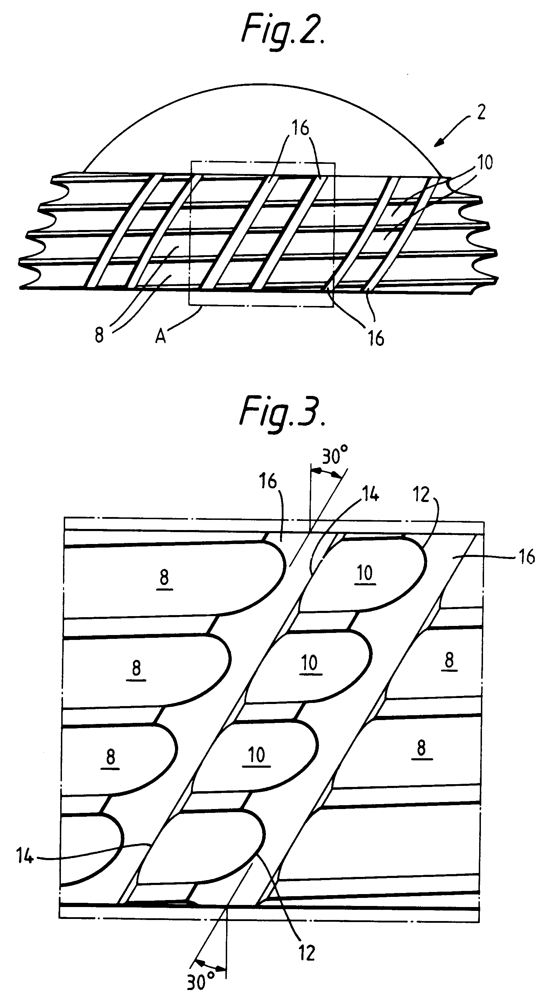

Figure 2 represents a side elevation of the metal cup shell of Figure 1; and
Figure 3 represents an enlarged view of section A in Figure 2.

The metal cup shell 2 may be fabricated from any suitably metal e.g. titanium or an alloy of titanium with aluminum and/or vanadium. The cup shell comprises a wall 4 which is may be hemispherical or frusto-conical defining a bore 6 into which a plastics liner (not shown) is generally inserted to define the spherical bearing surface for the ball portion of the hip joint.

The metal cup shell is provided on its outer surface with a self-tapping thread composed of alternating long thread segments 8 and short thread segments 10. Each thread segment has a thread radius which progressively decreases from the leading end 12 to the trailing end 14 of a segment. Thus in the drawing:

$\text{Ra > Rb > Rc > Rd}$

The length 'B' of the long segments is preferably between 1.5A and 18A where 'A' is the length of the short segments.

The width of each thread segment preferably decreases from the leading edge to the trailing edge.

The short and long thread segments are separated by tapping grooves 16, the total volume of the tapping grooves preferably exceeding the total volume of the thread segments. The tapping grooves 16 are inclined at an angle of 30° with respect to the axis of the cup shell.

The configuration of thread segments described above extends over the fully formed portion of the thread. The lead-in and lead-out portion of thread do not necessarily conform to this configuration. In particular, in the case of a hemispherical cup shell the lead end of the thread may start in the region of the pole of the hemisphere and the above configuration is not applicable since the thread radius increases in the lead portion.

## Claims

1. An acetabular implant of a hip joint prosthesis comprising a metal cup shell (2) having an external self-tapping thread extending around the circumference of the shell, the thread being divided into a plurality of segments (8, 10) by a plurality tapping grooves (16) characterised in that the thread radius (Ra, Rb, Rc, Rd) of thread segments progressively decreases from the leading end to the trailing end of a segment and that the tapping grooves (16) are arranged in pairs dividing the thread into alternate long (8) and short (10) segments.

2. An acetabular implant as claimed in claim 1 characterised in that the thread radius of a segment decreases from the leading end of the segment to the trailing end of the segment by at least 3% and not more than 30% of the depth of the thread segment.

3. An acetabular implant as claimed in claim 2 characterised in that the decrease is about 10%.

4. An acetabular implant as claimed in any preceding claim characterised in that the width of a thread segment progressively decreases from the leading end to the trailing end of the segment.

5. An acetabular implant as claimed in any preceding claim characterised in that the total volume of the thread tapping grooves is at least equal to the total volume of the thread segments.

6. An acetabular implant as claimed in any preceeding claim characterised in that the long thread segments (8) are from 50 to 1800% longer than the short thread segments (10).

7. An acetabular implant as claimed in claim 6 characterised in that the long thread segments (8) are 500% longer than the short thread segments (10).

## Patentansprüche

1. Acetabularimplantat einer Hüftgelenkprothese mit einer metallischen Pfannenschale (2), die ein sich um den Umfang der Schale erstreckendes selbstschneidendes Außengewinde aufweist, das durch Gewindeschneidnuten (16) in mehrere Segmente (8, 10) unterteilt ist, dadurch gekennzeichnet, daß der Gewinderadius (Ra, Rb, Rc, Rd) der Gewindesegmente vom vorderen zum hinteren Ende eines Segments progressiv abnimmt und daß die Gewindeschneidnuten (16) in Paaren vorgesehen sind, die das Gewinde in miteinander abwechselnde lange (8) und kurze Segmente (10) unterteilen.

2. Acetabularimplantat nach Anspruch 1, dadurch gekennzeichnet, daß der Gewinderadius eines Segments vom vorderen zum hinteren Ende des Segments um wenigstens 3% und nicht mehr als 30% der Tiefe des Gewindesegments abnimmt.

3. Acetabularimplantat nach Anspruch 2, dadurch gekennzeichnet, daß die Abnahme ungefähr 10% beträgt.

4. Acetabularimplantat nach einem der vorhergehenden Ansprüche, dadurch gekennzeichnet, daß die Breite eines Gewindesegments vom vorderen zum hinteren Ende des Segments progressiv abnimmt.

5. Acetabularimplantat nach einem der vorhergehenden Ansprüche, dadurch gekennzeichnet, daß das Gesamtvolumen der Gewindeschneidnuten mindestens gleich dem Gesamtvolumen der Gewindesegmente ist.

6. Acetabularimplantat nach einem der vorhergehenden Ansprüche, dadurch gekennzeichnet, daß die langen Gewindesegmente (8) zwischen 50 und 1800% länger sind als die kurzen Gewindesegmente (10).

7. Acetabularimplantat nach Anspruch 6, dadurch gekennzeichnet, daß die langen Gewindesegmente (8) 500% länger sind als die kurzen Gewindesegmente (10).

## Revendications

1. Implant acétabulaire d'une prothèse de l'articulation de la hanche, comportant une coque métallique (2) formant coupelle et présentant un filetage auto-taraudeur extérieur qui s'étend autour de la circonférence de la coque, le filetage étant divisé en une pluralité de segments (8, 10) par une pluralité de rainures de taraudage (16), implant caractérisé par le fait que le rayon de filetage (Ra, Rb, Rc, Rd) des segments du filetage décroît progressivement de l'extrémité avant à l'extrémité arrière d'un segment et par le fait que les rainures de taraudage (16) sont disposées par paires divisant le filetage alternativement en grands segments (8) et en petits segments (10).

2. Implant acétabulaire selon la revendication 1, caractérisé par le fait que le rayon de filetage d'un segment décroît de l'extrémité avant du segment l'extrémié arrière du segment d'au moins 3% et de pas plus de 30% de la profondeur du segment de filetage.

3. Implant acétabulaire selon la revendication 2, caractérisé par le fait que la décroissance est d'environ 10%.

4. Implant acétabulaire selon l'une quelconque des revendications précédentes, caractérisé par le fait que la largeur d'un segment de filetage décroît progressivement de l'extrémité avant à l'extrémtité arrière du segment.

5. Implant acétabulaire selon l'une quelconque des revendications précédentes, caractérisé par le fait que le volume total des rainures de taraudage du filetage est au moins égal au volume total des segments de filetage.

6. Implant acétabulaire selon l'une quelconque des revendications précédentes, caractérisé par le fait que les grands segments de filetage (8) sont de 50 à 1800% plus grands que les petits segments de filetage (10).

7. Implant acétabulaire selon la revendication 6, caractérisé par le fait que les grands segments de filetage (8) sont 500% plus grands que les petits segments de filetage (10).
